# EUROPEAN PATENT APPLICATION

(11) **EP 3 042 600 A1**
(43) Date of publication of application: **13.07.2016**
(21) Application number: 14841660.5
(22) Date of filing: 02.09.2014
(51) Int. Cl.: A61B 1/00

(54) **OPTICAL MEASUREMENT DEVICE**

(30) Priority: 03.09.2013 US 201361873027 P
(71) Applicant: OLYMPUS CORPORATION, Shibuya-ku Tokyo 151-0072 (JP)
(72) Inventor: TORIYAMA, Seiki, Tokyo 151-0072 (JP); AKUI, Nobuaki, Tokyo 151-0072 (JP)
(74) Representative: Gunzelmann, Rainer
(86) International application number: PCT/JP2014/073024
(87) International publication number: WO 2015/033915

(57) **Abstract**

An optical measurement apparatus 2 includes an imaging unit 23 and a partition unit 24. The imaging unit 23 receives rays of light transmitted from exit ends T1 of a first light receiving fiber 56, a second light receiving fiber 57, and a third light receiving fiber 58 of a measurement probe 5 and performs photoelectric conversion in order to generate and output an electric signal. The partition unit 24 optically isolates paths for the rays of light transmitted from the exit ends T1 of the first light receiving fiber 56, the second light receiving fiber 57, and the third light receiving fiber 58 of the measurement probe 5, from one another in a space from an end surface of the measurement probe to a light reception surface T3 of the imaging unit 23.

## Description

### Field

The present invention relates to an optical measurement apparatus for measuring optical characteristics of body tissues.

### Background

In recent years, there have been optical measurement apparatuses known to irradiate body tissues with illumination light for estimating properties of the body tissues on the basis of measurement values of detection light reflected or scattered from the body tissues. Such an optical measurement apparatus is used in combination with an endoscope for observing organs, such as digestive organs. As this type of optical measurement apparatus, an optical measurement apparatus using low-coherence enhanced backscattering (LEBS) has been proposed for detecting properties of body tissues by irradiating the body tissues with low-coherent white light having a short spatial coherence length from an illumination fiber of a measurement probe, by detecting scattered light incident at mutually different angles with a plurality of light receiving fibers, and by measuring the intensity distribution of the scattered rays of light with spectroscopes provided at the light receiving fibers (see Patent Literature 1).

There is a technique known to allow one imaging element to receive rays of light transmitted from a plurality of light receiving fibers for detecting the properties of body tissues on the basis of a result of the received rays of light (see Patent Literature 2). In this technique, the rays of light transmitted from the exit ends of the light receiving fibers are each dispersed by spectroscopic elements, such as diffraction gratings or prisms, to prevent the rays of light from overlapping on the light reception surface of the imaging element, and thereby to allow the one imaging element to detect the properties of the body tissues.

### Citation List

### Patent Literature

Patent Literature 1: Japanese Patent No. 5049415
Patent Literature 2: Japanese Laid-open Patent Publication No. 2011-005235

### Summary

### Technical Problem

Patent Literature 1 described above has difficulty in downsizing the optical measurement apparatus because the light receiving fibers need to be each provided with a spectroscope, which necessitates the provision of spaces for placing the spectroscopes in the optical measurement apparatus.

The use of one imaging element, in place of the plurality of spectroscopes, may be contemplated for the downsizing of the optical measurement apparatus. Patent Literature 2 described above, however, has a drawback of increased complexity in the configuration of the optical measurement apparatus, since accurate dispersion of the rays of light transmitted from the exit ends of the plurality of light receiving fibers necessitates to make certain that the spectroscopic elements are each located at a specified position between the plurality of light receiving fibers and the imaging element.

The present invention has been made in view of the foregoing, and an object of the invention is to provide an optical measurement apparatus that allows further downsizing through a simple configuration.

### Solution to Problem

In order to solve the above-mentioned problem and to achieve the object, an optical measurement apparatus according to the invention is an optical measurement apparatus to which a measurement probe is configured to be detachably attached. The measurement probe has at an end surface thereof exit ends of a plurality of optical fibers. The optical measurement apparatus includes: an imaging unit configured to receive rays of light transmitted from the exit ends of the plurality of optical fibers and to perform photoelectric conversion in order to generate and output an electric signal; and a partition unit configured to optically isolate paths for the rays of light transmitted from the exit ends of the plurality of optical fibers, from one another in a space from the end surface to a light reception surface of the imaging unit.

According to the optical measurement apparatus of the invention, in the above-described invention, the partition unit comes into contact with the end surface at one end of the partition unit, and comes into contact with the light reception surface of the imaging unit at another end of the partition unit.

In the above-described invention, the optical measurement apparatus according to the invention further includes a connector unit into which the measurement probe is configured to be inserted. The partition unit is provided between the connector unit and the imaging unit, and keeps a constant distance between the end surface and the light reception surface when the measurement probe is inserted into the connector unit.

In the above-described invention, the optical measurement apparatus according to the invention further includes: a support member configured to support the imaging unit; and an energizing member configured to energize the support member toward the end surface.

According to the optical measurement apparatus of the invention, in the above-described invention, the imaging unit includes a plurality of imaging elements, and the plurality of imaging elements is provided on the paths for the rays of light isolated by the partition unit.

In the above-described invention, the optical measurement apparatus according to the invention further includes a connector unit into which the measurement probe is configured to be inserted. The connector unit keeps a constant distance between the end surface and the light reception surface when the measurement probe is inserted. Advantageous Effects of Invention

In an optical measurement apparatus according to this invention, a partition unit optically isolates paths for rays of light transmitted from exit ends of a plurality of optical fibers of a measurement probe, from one another in a space from an end surface of the measurement probe to a light reception surface of an imaging unit, and thus the optical measurement apparatus can be downsized through the simple configuration.

### Brief Description of Drawings

FIG. 1 is a diagram in outline of a configuration of an optical measurement system according to a first embodiment of the invention.
FIG. 2 is a schematic block diagram of a functional configuration of the optical measurement system according to the first embodiment of the invention.
FIG. 3 is a schematic sectional view of a configuration of a light source unit, a connector unit, an imaging unit, and a partition unit of an optical measurement apparatus and a proximal end portion of a measurement probe according to the first embodiment of the invention.
FIG. 4 is a schematic perspective view of a relationship of the light source unit, the imaging unit, and the partition unit of the optical measurement apparatus and optical fibers of the measurement probe according to the first embodiment of the invention.
FIG. 5 is a diagram of the optical measurement system according to the first embodiment of the invention used with an endoscope system.
FIG. 6 is a schematic sectional view of a configuration of a connector unit of an optical measurement apparatus and a measurement probe according to a second embodiment of the invention.
FIG. 7 is a schematic perspective view of a configuration of a partition unit of the optical measurement apparatus according to a first modification of the second embodiment of the invention.
FIG. 8 is a schematic perspective view of a configuration of a partition unit of the optical measurement apparatus according to a second modification of the second embodiment of the invention.
FIG. 9 is a schematic perspective view of a configuration of a partition unit of the optical measurement apparatus according to a third modification of the second embodiment of the invention.
FIG. 10 is a schematic sectional view of a configuration of a connector unit of an optical measurement apparatus according to a third embodiment of the invention.
FIG. 11 is a schematic perspective view of a configuration of an imaging unit and a partition unit according to a fourth embodiment of the invention.
FIG. 12 is a schematic perspective view of a configuration of an imaging unit and a partition unit according to a first modification of the fourth embodiment of the invention.

### Description of Embodiments

Modes for carrying out the invention (hereinafter, referred to as "embodiment(s)") will now be described below with reference to the drawings. The same reference signs are used to designate the same elements throughout the drawings. The drawings are schematic and that the relationship between a thickness and a width of each material, a ratio of materials, and the like may be different from those in reality. Dimensional relationships and ratios may be different between the drawings. Embodiments should not be construed to limit the invention.

### (First Embodiment)

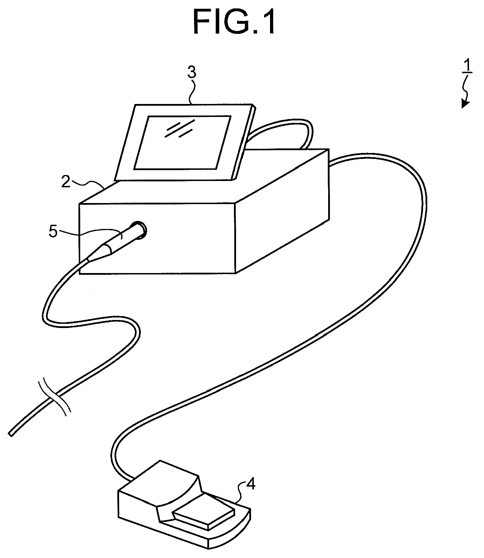

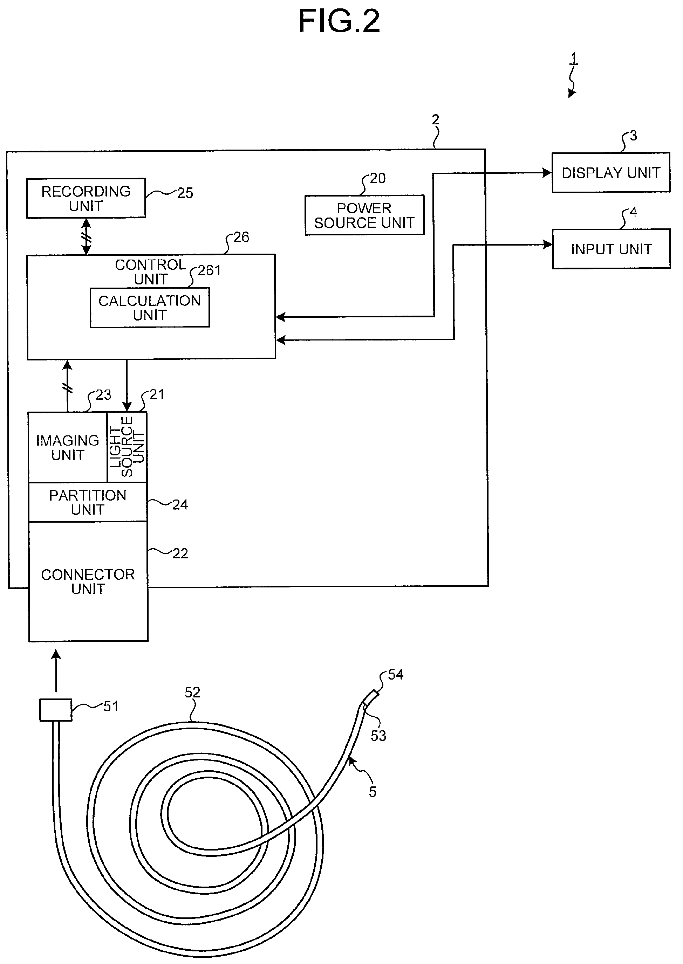

FIG. 1 is a diagram in outline of a configuration of an optical measurement system according to a first embodiment of the invention. FIG. 2 is a schematic block diagram of a functional configuration of the optical measurement system according to the first embodiment of the invention.

An optical measurement system 1 illustrated in FIGS. 1 and 2 includes an optical measurement apparatus 2 that optically measures a measurement target, such as a body tissue, which is a scatterer, to detect properties (characteristics) of the measurement target, a display unit 3 that displays a result of the measurement by the optical measurement apparatus 2, an input unit 4 that receives an input of an instruction signal to instruct the optical measurement apparatus 2 to perform the measurement, and a measurement probe 5 that can be attached to and detached from the optical measurement apparatus 2 and is to be inserted into a subject.

A configuration of the optical measurement apparatus 2 will now be described. The optical measurement apparatus 2 includes a power source unit 20, a light source unit 21, a connector unit 22, an imaging unit 23, a partition unit 24, a recording unit 25, and a control unit 26. The power source unit 20 supplies electric power to each unit of the optical measurement apparatus 2.

The light source unit 21 transmits illumination light through the connector unit 22 to the measurement probe 5. The light source unit 21 is realized by using a light-emitting element, which is an incoherent light source, such as a white light emitting diode (LED), together with one or more lenses as needed. Examples of such a lens include a condenser lens and a collimating lens. The light source unit 21 transmits incoherent light, having at least one spectral component, in the form of illumination light through the partition unit 24 and the connector unit 22 to the measurement probe 5. Alternatively, the light source unit 21 may be realized by using an incoherent light source, such as a xenon lamp, a tungsten lamp, and a halogen lamp.

The connector unit 22 detachably connects the measurement probe 5 to the optical measurement apparatus 2. The connector unit 22 is realized by using, for example, an SMA (Sub-Miniature Type A) connector.

The imaging unit 23 receives return light of the illumination light, which has been transmitted from a distal end of the measurement probe 5 and reflected and/or scattered from the measurement target, and performs photoelectric conversion to generate an electric signal. The imaging unit 23 outputs the signal to the control unit 26. The imaging unit 23 is realized by using an imaging element, such as a charge coupled device (CCD) and a complementary metal oxide semiconductor (CMOS).

The partition unit 24 optically isolates paths for rays of light transmitted from exit ends of a plurality of optical fibers of the measurement probe 5, from one another in a space from an end surface of the measurement probe 5 to a light reception surface of the imaging unit 23. Specifically, the light reception surface, through which the imaging unit 23 receives the rays of light transmitted from the exit ends of the plurality of optical fibers of the measurement probe 5, is partitioned into a plurality, with the rays of light transmitted from the exit ends of the plurality of optical fibers of the measurement probe 5 optically separated.

The recording unit 25 records various programs for operating the optical measurement apparatus 2 and various types of data and parameters to be used by the optical measurement apparatus 2. The recording unit 25 is realized by using a volatile memory, a nonvolatile memory, or the like. The recording unit 25 temporarily records information and data during processing of the optical measurement apparatus 2. The recording unit 25 also records the results of the measurement by the optical measurement apparatus 2. Here, the recording unit 25 may be configured with a memory card to be attached from the outside of the optical measurement apparatus 2.

The control unit 26 controls a processing operation of each unit of the optical measurement apparatus 2. The control unit 26 is configured with a central processing unit (CPU) or the like and provides centralized control of the optical measurement apparatus 2 by transferring instruction information, data, and the like to each unit of the optical measurement apparatus 2. The control unit 26 includes a calculation unit 261.

The calculation unit 261 performs more than one type of operation processing on the basis of the electric signal input from the imaging unit 23 to calculate a characteristic value on the property of the measurement target.

The display unit 3 outputs various types of information of the optical measurement apparatus 2. Specifically, the display unit 3 displays information input from the optical measurement apparatus 2. The display unit 3 is realized by using a display panel, such as liquid crystal and organic electro luminescence (EL), a speaker, and the like. Here, a touch panel may be provided on a display screen of the display unit 3 for receiving an input of a position signal corresponding to a position of a touch from the outside.

The input unit 4 receives an input of an instruction signal to instruct the optical measurement apparatus 2 to perform the measurement. The input unit 4 is realized by using an input interface, such as a foot switch, a keyboard, and a mouse.

The measurement probe 5 is configured with at least the plurality of optical fibers. Specifically, the measurement probe 5 is realized by using an illumination fiber (an illumination channel) that transmits the illumination light to the measurement target and a plurality of light receiving fibers (light reception channels) that rays of return light of the illumination light reflected and/or scattered from the measurement target enter at different angles. The measurement probe 5 includes a proximal end portion 51 that is detachably connected to the connector unit 22 of the optical measurement apparatus 2, a flexible portion 52 having flexibility, and a distal end portion 53 that transmits the illumination light supplied through the connector unit 22 from the light source unit 21 and receives the returned illumination light from the measurement target. The distal end portion 53 is provided with a rod lens 54 that maintains a constant distance from the measurement target to the distal end portion 53.

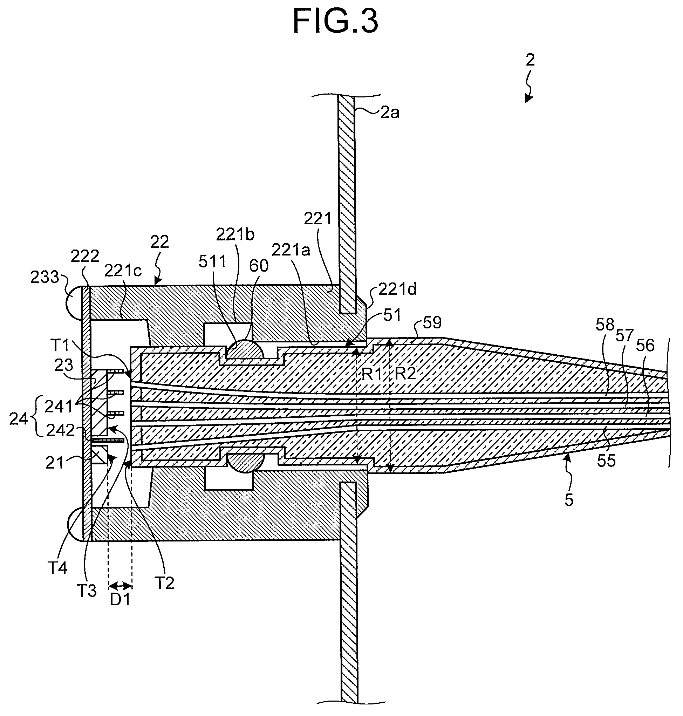

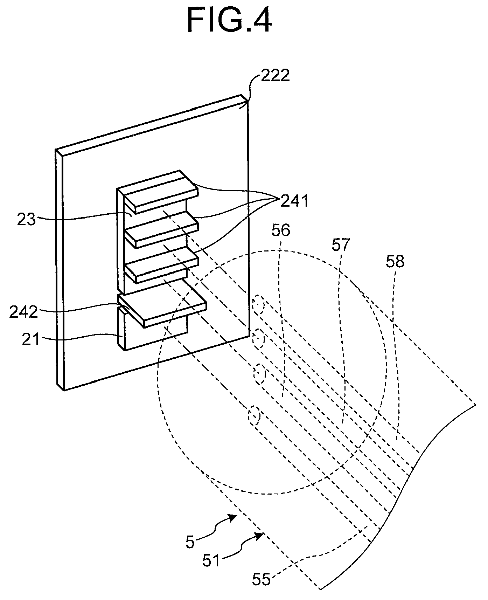

A configuration of the light source unit 21, the connector unit 22, the imaging unit 23, the partition unit 24, and the proximal end portion 51 of the measurement probe 5 will now be described in detail. FIG. 3 is a schematic sectional view of an arrangement of the light source unit 21, the connector unit 22, the imaging unit 23, the partition unit 24, and the proximal end portion 51 of the measurement probe 5. FIG. 4 is a schematic perspective view of a relationship of the light source unit 21, the imaging unit 23, the partition unit 24, and the optical fibers of the measurement probe 5.

The measurement probe 5 will now be described. The measurement probe 5 includes the proximal end portion 51 to be detachably inserted into the connector unit 22, an illumination fiber 55, a first light receiving fiber 56, a second light receiving fiber 57 and a third light receiving fiber 58, a cylindrical holding portion 59 that holds the illumination fiber 55, the first light receiving fiber 56, the second light receiving fiber 57, and the third light receiving fiber 58 therein, and an annular pressing member 60. The illumination fiber 55 propagates the illumination light, supplied from the light source unit 21 through the connector unit 22 and the partition unit 24, to the distal end portion 53 of the measurement probe 5 and transmits the illumination light onto the measurement target. The first light receiving fiber 56, the second light receiving fiber 57 and the third light receiving fiber 58 propagate the rays of return light of the illumination light, reflected and/or scattered from the measurement target and entering through the distal end portion 53 at mutually different angles, to the proximal end portion 51.

The proximal end portion 51 is inserted into the connector unit 22 detachably. An outer diameter R1 of the proximal end portion 51 is formed smaller than an outer diameter R2 of the holding portion 59. The proximal end portion 51 has a groove portion 511 notched therein annularly toward a center thereof.

The pressing member 60 is attached to the proximal end portion 51 in the groove portion 511. The pressing member 60 is realized by using a C ring spring and the like, which is capable of elastic deformation in a radial direction.

The connector unit 22 will now be described. The connector unit 22 includes a connector frame 221, provided at a casing 2a of the optical measurement apparatus 2, and a support member 222 that supports the light source unit 21, the imaging unit 23, and the partition unit 24.

The connector frame 221 is substantially tubular. The connector frame 221 has an insertion portion 221a. The insertion portion 221a retains the proximal end portion 51 of the measurement probe 5. The connector frame 221 has a first groove portion 221b and a second groove portion 221c notched therein annularly from the inner periphery side toward the outer periphery side of the insertion portion 221a. The connector frame 221 also has an abutment portion 221d exposed from an outer portion of the casing 2a and abutting on part of the proximal end portion 51 of the measurement probe 5.

The abutment portion 221d sets a constant distance from the light reception surface of the imaging unit 23 to the end surface of the measurement probe 5 when the proximal end portion 51 of the measurement probe 5 is inserted into the insertion portion 221a. Specifically, when the proximal end portion 51 of the measurement probe 5 is inserted into the insertion portion 221a of the connector frame 221, the abutment portion 221d comes into contact with the holding portion 59 to set a distance, from exit ends T1 (outgoing surfaces) of the first light receiving fiber 56, the second light receiving fiber 57, and the third light receiving fiber 58 and an incoming end T2 (an incoming surface) of the illumination fiber 55 at the proximal end portion 51 of the measurement probe 5, to a light reception surface T3 (a light reception region) of the imaging unit 23 and a light emission surface T4 (a light emission region) of the light source unit 21 to a distance D1.

The support member 222 supports the light source unit 21, the imaging unit 23, and the partition unit 24. Specifically, the support member 222 supports the light emission surface T4 of the light source unit 21 and the light reception surface T3 of the imaging unit 23 to face the insertion side at which the measurement probe 5 is to be inserted. The support member 222 is secured to the connector frame 221 with a screw 233 or the like such that the light source unit 21, the imaging unit 23, and the partition unit 24 are accommodated in the second groove portion 221c of the connector frame 221.

The light source unit 21 is rectangular as illustrated in FIG. 4. The light source unit 21 is supported by the support member 222 such that the light emission surface T4 faces the incoming end T2 of the measurement probe 5. The light source unit 21 emits the illumination light under the control of the control unit 26 to supply the illumination light to the illumination fiber 55 of the measurement probe 5.

The imaging unit 23 is rectangular as illustrated in FIG. 4. The imaging unit 23 is supported by the support member 222 such that the light reception surface T3 faces the exit ends T1 (the end surface) of the measurement probe 5. The imaging unit 23 performs the photoelectric conversion on the rays of light transmitted from the exit ends T1 of the first light receiving fiber 56, the second light receiving fiber 57, and the third light receiving fiber 58 of the measurement probe 5 to generate an electric signal and outputs the electric signal to the control unit 26.

The partition unit 24 includes first partition sections 241 and a second partition section 242 as illustrated in FIGS. 3 and 4.

The first partition sections 241 optically isolate the paths for the rays of light transmitted from the exit ends T1 of the first light receiving fiber 56, the second light receiving fiber 57, and the third light receiving fiber 58 of the measurement probe 5, from one another in the space from the end surface of the measurement probe 5 to the light reception surface T3 of the imaging unit 23. Specifically, the first partition sections 241 partition the light reception surface T3, through which the imaging unit 23 receives the rays of light, into the plurality, with the rays of light transmitted from the exit ends T1 of the first light receiving fiber 56, the second light receiving fiber 57, and the third light receiving fiber 58 of the measurement probe 5 optically isolated. The first partition sections 241 are realized by using light-blocking members. The first partition sections 241 are shaped into plates with a length to set a constant distance to the exit ends T1 of the first light receiving fiber 56, the second light receiving fiber 57, and the third light receiving fiber 58. The first partition sections 241 are fixed with ends thereof in contact with the light reception surface T3 of the imaging unit 23.

The second partition section 242 prevents the imaging unit 23 from receiving the illumination light emitted by the light source unit 21. The second partition section 242 is realized by using a light-blocking member. The second partition section 242 is shaped into a plate with a length to set a constant distance from the light emission surface T4 of the light source unit 21 to the incoming end T2 of the illumination fiber 55. The second partition section 242 is fixed with an end thereof in contact with the support member 222 and provided between the light source unit 21 and the imaging unit 23.

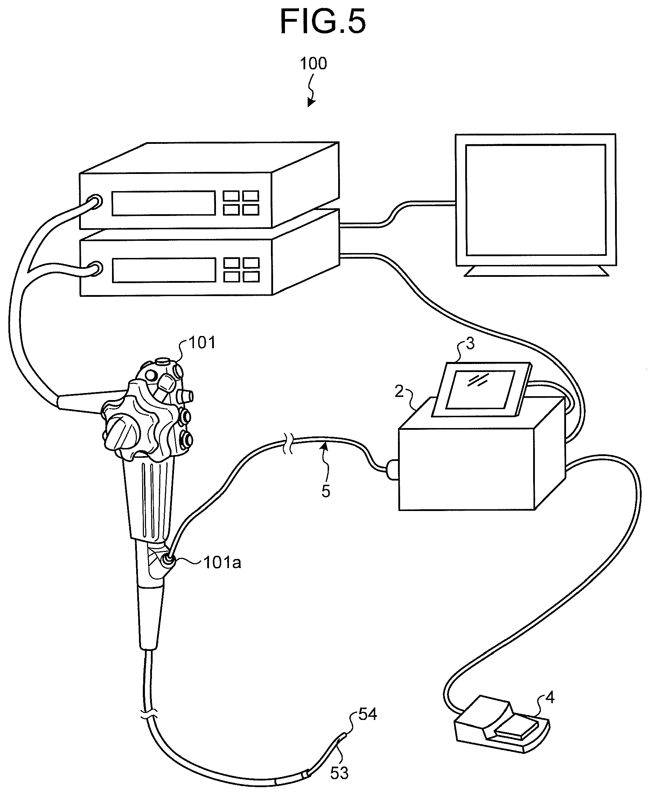

The optical measurement system 1 configured as described above allows the measurement probe 5 to be inserted, as illustrated in FIG. 5, through a treatment tool channel 101a provided in an endoscope apparatus 101 (an endoscope scope) of an endoscope system 100, so that the illumination fiber 55 transmits the illumination light onto the measurement target and that the first light receiving fiber 56, the second light receiving fiber 57, and the third light receiving fiber 58 transmit the rays of return light of the illumination light, reflected and/or scattered from the measurement target and received through the rod lens 54 by the distal end portion 53, through the exit ends T1 to the light reception surface T3 of the imaging unit 23 partitioned by the partition unit 24. The calculation unit 261 then computes the characteristic value of the property of the measurement target on the basis of the result of the measurement of the light reception surface T3 of the imaging unit 23.

When the proximal end portion 51 of the measurement probe 5 is inserted into the connector unit 22, the pressing member 60 is inserted as compressed toward the center side to have an identical diameter to that of the insertion portion 221a of the connector frame 221. The pressing member 60 then extends in the radial direction when reaching the first groove portion 221b of the connector frame 221. The pressing member 60 at this point in time expands in the radial direction with the pressing member 60 in contact with a corner portion of the first groove portion 221b, and thus the pressing member 60 travels in a direction of the imaging unit 23. Since the pressing member 60 is attached to the groove portion 511, the pressing member 60 pushes the proximal end portion 51 in the direction of the imaging unit 23 while traveling in the direction of the imaging unit 23, so that the proximal end portion 51 is moved into a position in which a step portion formed by the outer diameters R1 and R2 abuts on 221d. This allows the proximal end portion 51 to be attached to the connector unit 22. As a result, a user can mount the measurement probe 5 on the optical measurement apparatus 2 with one operation.

In the first embodiment according to the invention described above, the partition unit 24 optically isolates the paths for the rays of light transmitted from the exit ends T1 of the first light receiving fiber 56, the second light receiving fiber 57, and the third light receiving fiber 58 of the measurement probe 5, from one another in the space from the end surface of the measurement probe 5 to the light reception surface T3 of the imaging unit 23, and thus, the return light transmitted from the first light receiving fiber 56, the second light receiving fiber 57, and the third light receiving fiber 58 can be detected with one piece of the imaging unit 23 alone. As a result, the optical measurement apparatus 2 can be downsized with the simple configuration.

Further, in the first embodiment according to the invention, the rays of light transmitted from the exit ends T1 of the first light receiving fiber 56, the second light receiving fiber 57, and the third light receiving fiber 58 are prevented from being detected as a disturbance (noise) by another of the light reception surface T3 of the imaging unit 23, allowing the property of the measurement target to be measured accurately.

Further, in the first embodiment according to the invention, when the proximal end portion 51 of the measurement probe 5 is inserted into the insertion portion 221a of the connector unit 22, the abutment portion 221d of the connector unit 22 comes into contact with the measurement probe 5, setting the distance from the exit ends T1 of the proximal end portion 51 of the measurement probe 5 to the light reception surface T3 of the imaging unit 23 to the distance D1. As a result, an effect of diffused reflection due to an excessively short distance from the light reception surface T3 of the imaging unit 23 to the end surface of the measurement probe 5 can be prevented, and a reduction in detection efficiency due to an excessively long distance from the light reception surface T3 of the imaging unit 23 to the end surface of the measurement probe 5 can be prevented.

### (Second Embodiment)

A second embodiment of the invention will now be described. An optical measurement apparatus according to the second embodiment differs from the optical measurement apparatus according to the first embodiment described above in shape of the connector unit, the partition unit, and the proximal end portion of the measurement probe. Hence, a connector unit and a partition unit of the optical measurement apparatus and a proximal end portion of a measurement probe will be described hereinafter. The same reference signs are used to designate the same elements as those in the first embodiment described above.

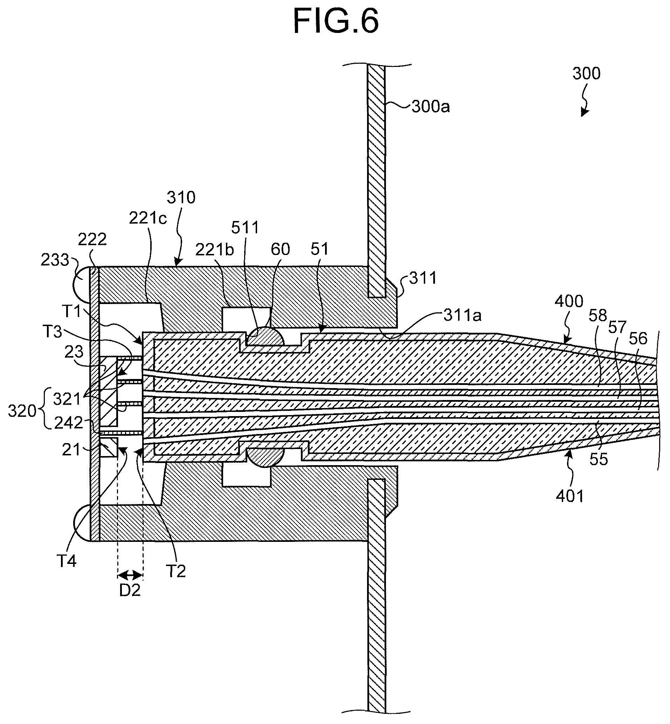

FIG. 6 is a schematic sectional view of a configuration of a connector unit 310 of an optical measurement apparatus 300 and a measurement probe 400 according to the second embodiment of the invention.

The measurement probe 400 will now be described. As illustrated in FIG. 6, the measurement probe 400 includes a proximal end portion 51, an illumination fiber 55, a first light receiving fiber 56, a second light receiving fiber 57, a third light receiving fiber 58, a pressing member 60, and a holding portion 401.

The holding portion 401 is cylindrical and holds the illumination fiber 55, the first light receiving fiber 56, the second light receiving fiber 57, and the third light receiving fiber 58. The holding portion 401 and the proximal end portion 51 are integrated to extend continuously.

The optical measurement apparatus 300 will now be described. The optical measurement apparatus 300 includes a light source unit 21, an imaging unit 23, the connector unit 310, and a partition unit 320.

The connector unit 310 includes a support member 222 and a connector frame 311 provided at a casing 300a of the optical measurement apparatus 300. The connector frame 311 is substantially tubular. The connector frame 311 has an insertion portion 311a. The connector frame 311 has a first groove portion 221b and a second groove portion 221c formed therein.

The partition unit 320 includes a second partition section 242 and first partition sections 321. The first partition sections 321 optically isolate paths for rays of light transmitted from exit ends T1 of the first light receiving fiber 56, the second light receiving fiber 57, and the third light receiving fiber 58 of the measurement probe 400, from one another in a space from an end surface of the measurement probe 400 to a light reception surface T3 of the imaging unit 23. Specifically, the first partition sections 321 isolate the light reception surface T3, through which the imaging unit 23 receives the rays of light, into a plurality, with the rays of light transmitted from the exit ends T1 of the first light receiving fiber 56, the second light receiving fiber 57, and the third light receiving fiber 58 of the measurement probe 5 optically separated.

When the measurement probe 400 is inserted into the insertion portion 311a of the connector unit 310, the first partition sections 321 set a distance from the light reception surface T3 of the imaging unit 23 to the exit ends T1 of the first light receiving fiber 56, the second light receiving fiber 57, and the third light receiving fiber 58 to a distance D2. The first partition sections 321 are realized by using light-blocking members. The first partition sections 321 are shaped into rectangles with a length to set the distance to the exit ends T1 of the first light receiving fiber 56, the second light receiving fiber 57, and the third light receiving fiber 58 to the distance D2.

The first partition sections 321 are fixed with ends thereof in contact with the light reception surface T3 of the imaging unit 23. The first partition sections 321 also come into contact at other ends with the exit ends T1 of the measurement probe 400 when the measurement probe 400 is inserted into the insertion portion 221a of the connector frame 311. Here, the first partition sections 321 and the second partition section 242 may be secured to the support member 222 side or may be secured to the measurement probe 400 side.

In the second embodiment according to the invention described above, the partition unit 320 optically isolates the paths for the rays of light transmitted from the exit ends T1 of the first light receiving fiber 56, the second light receiving fiber 57, and the third light receiving fiber 58 of the measurement probe 400, from one another in the space from the end surface of the measurement probe 400 to the light reception surface T3 of the imaging unit 23. Further, the partition unit 320 comes into contact with the end surface of the measurement probe 400 when the measurement probe 400 is inserted into the connector unit 310. Thus, the return light transmitted from the first light receiving fiber 56, the second light receiving fiber 57, and the third light receiving fiber 58 can be detected with one piece of the imaging unit 23 alone. As a result, the optical measurement apparatus 300 can be downsized with the simple configuration.

Further, in the second embodiment according to the invention, the rays of light transmitted from the exit ends of the first light receiving fiber 56, the second light receiving fiber 57, and the third light receiving fiber 58 are prevented from being detected as a disturbance (noise) by another of the light reception surface T3 of the imaging unit 23, allowing the property of the measurement target to be measured accurately.

Further, in the second embodiment according to the invention, when the proximal end portion 51 of the measurement probe 400 is inserted into the insertion portion 311a of the connector unit 310, the partition unit 320 sets the distance from the exit ends T1 of the proximal end portion 51 of the measurement probe 400 to the light reception surface T3 of the imaging unit 23 to the distance D2. Thus, an effect of diffused reflection due to an excessively short distance from the light reception surface T3 of the imaging unit 23 to the end surface of the measurement probe 5 can be prevented, and a reduction in detection efficiency due to an excessively long distance from the light reception surface T3 of the imaging unit 23 to the end surface of the measurement probe 400 can be prevented.

Further, in the second embodiment according to the invention, the step portion formed by the outer diameters R1 and R2 for positioning the proximal end portion 51 described in the first embodiment is precluded, and thus the measurement probe 400 can be manufactured with ease.

### (First Modification of Second Embodiment)

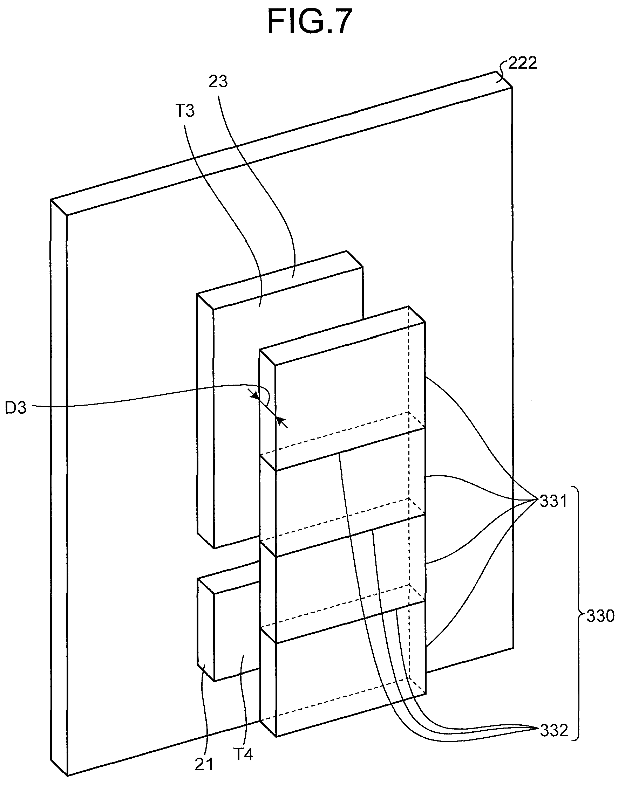

In the second embodiment of the invention, the shape of the partition unit may be changed. FIG. 7 is a schematic perspective view of a configuration of a partition unit according to a first modification of the second embodiment of the invention.

A partition unit 330 illustrated in FIG. 7 optically isolates the paths for the rays of light transmitted from the exit ends T1 of the first light receiving fiber 56, the second light receiving fiber 57, and the third light receiving fiber 58 of the measurement probe 400, from one another in the space from the end surface of the measurement probe 400 to the light reception surface T3 of the imaging unit 23. Specifically, the partition unit 330 partitions the light reception surface T3, through which the imaging unit 23 receives the rays of light, into the plurality, with the rays of light transmitted from the exit ends of the first light receiving fiber 56, the second light receiving fiber 57, and the third light receiving fiber 58 of the measurement probe 400 optically separated. The partition unit 330 also sets a distance from the light reception surface T3 of the imaging unit 23 to the exit ends of the first light receiving fiber 56, the second light receiving fiber 57, and the third light receiving fiber 58 to a constant distance D2.

The partition unit 330 is realized by using transparent members and light-blocking members, which have a plate shape and have a specified thickness D3. Specifically, the partition unit 330 is configured with transparent members 331 such as glass and plastic, and light-blocking members 332 for blocking light. A light-blocking member 332 is provided between a transparent member 331 and a transparent member 331. The transparent members 331 and the light-blocking members 332 are integrated with an adhesive or the like. Alternatively, the partition unit 330 may be formed by integrating the transparent members 331 and the light-blocking members 332 through two-color molding.

The first modification of the second embodiment according to the invention as described above achieves a similar effect to the second embodiment described above and can downsize the optical measurement apparatus 300 with the simple configuration.

### (Second Modification of Second Embodiment)

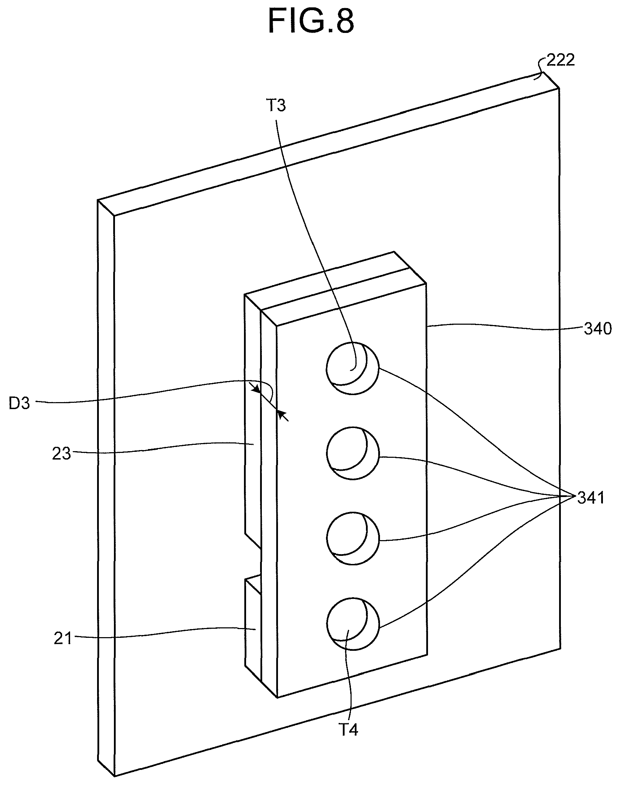

FIG. 8 is a schematic perspective view of a configuration of a partition unit according to a second modification of the second embodiment of the invention.

A partition unit 340 illustrated in FIG. 8 optically isolates the paths for the rays of light transmitted from the exit ends T1 of the first light receiving fiber 56, the second light receiving fiber 57, and the third light receiving fiber 58 of the measurement probe 400, from one another in the space from the end surface of the measurement probe 400 to the light reception surface T3 of an imaging unit 23. Specifically, the partition unit 340 partitions the light reception surface T3, through which the imaging unit 23 receives the rays of light, into the plurality, with the rays of light transmitted from the exit ends T1 of the first light receiving fiber 56, the second light receiving fiber 57, and the third light receiving fiber 58 of the measurement probe 400 optically separated. The partition unit 340 also sets a distance from the light reception surface T3 of the imaging unit 23 to the exit ends T1 of the first light receiving fiber 56, the second light receiving fiber 57, and the third light receiving fiber 58 to a constant distance. Specifically, the partition unit 340 has holes 341 therethrough at positions corresponding to the exit ends T1 of the first light receiving fiber 56, the second light receiving fiber 57, and the third light receiving fiber 58 and the incoming end T2 of the illumination fiber 55. The partition unit 340 is realized by using a plate-shaped light-blocking member having the specified thickness D3.

The second modification of the second embodiment according to the invention as described above achieves a similar effect to the second embodiment described above and can downsize the optical measurement apparatus 300 with the simple configuration.

### (Third Modification of Second Embodiment)

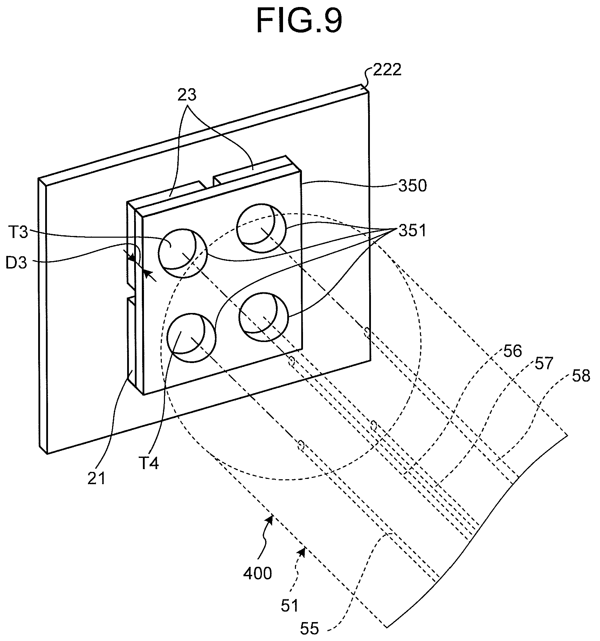

FIG. 9 is a schematic perspective view of a configuration of a partition unit according to a third modification of the second embodiment of the invention.

A partition unit 350 illustrated in FIG. 9 optically isolates the paths for the rays of light transmitted from the exit ends T1 of the first light receiving fiber 56, the second light receiving fiber 57, and the third light receiving fiber 58 of the measurement probe 400, from one another in the space from the end surface of the measurement probe 400 to the light reception surface T3 of the imaging unit 23. Specifically, the partition unit 350 has holes 351 therethrough at positions corresponding to the exit ends T1 of the first light receiving fiber 56, the second light receiving fiber 57, and the third light receiving fiber 58 and the light emission surface T4 of the light source unit 21. The partition unit 350 is realized by using a plate-shaped light-blocking member having a specified thickness D3. Further, the partition unit 350 has the holes 351 formed in correspondence with the positions of the light source unit 21 and the imaging unit 23.

The third modification of the second embodiment according to the invention as described above achieves a similar effect to the second embodiment described above and can downsize the optical measurement apparatus 300 with the simple configuration.

### (Third Embodiment)

A third embodiment of the invention will now be described. An optical measurement system according to the third embodiment differs from the optical measurement apparatus according to the second embodiment described above in shape of the connector unit. Hence, a connector unit of an optical measurement apparatus will be described hereinafter. The same reference signs are used to designate the same elements as those in the second embodiment described above.

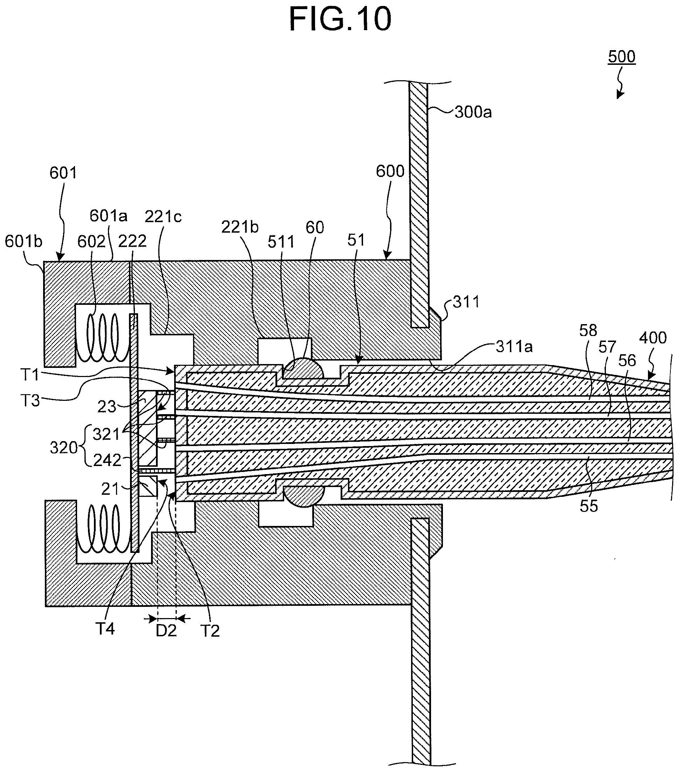

FIG. 10 is a schematic sectional view of a configuration of the connector unit of the optical measurement apparatus according to the third embodiment of the invention. A connector unit 600 illustrated in FIG. 10 includes a connector frame 311, a seat 601, and an energizing member 602.

The seat 601 has a substantially annular main portion 601a and an arm portion 601b extending from an inner periphery of the main portion 601a toward a center thereof. The main portion 601a and the arm portion 601b are integrated.

The energizing member 602 is provided between the arm portion 601b and a support member 222 and energizes the support member 222 toward the side from which a measurement probe 400 is inserted. The energizing member 602 is fixed at its one end to the arm portion 601b and at the other end to the support member 222. The energizing member 602 is realized by using a compression coil spring or the like.

An optical measurement apparatus 500 configured as described above allows a proximal end portion 51 of the measurement probe 400 to come into contact with a partition unit 320 when the proximal end portion 51 of the measurement probe 400 is inserted into an insertion portion 311a. At this point in time, the energizing member 602 energizes the support member 222 toward the measurement probe 400 so that a distance from exit ends T1 of a first light receiving fiber 56, a second light receiving fiber 57, and a third light receiving fiber 58 of the measurement probe 400 to a light reception surface T3 of an imaging unit 23 can be set to a distance D2 with certainty. The energizing member 602 at the same time can alleviate an impact to be applied to the imaging unit 23 from the contacting between the proximal end portion 51 and the partition unit 320.

In the third embodiment according to the invention described above, the partition unit 320 optically isolates paths for rays of light transmitted from the exit ends T1 of the first light receiving fiber 56, the second light receiving fiber 57, and the third light receiving fiber 58 of the measurement probe 400, from one another in a space from an end surface of the measurement probe 400 to the light reception surface T3 of the imaging unit 23. Further, the partition unit 320 comes into contact with the end surface of the measurement probe 400 when the measurement probe 400 is inserted into the connector unit 310. Thus, the return light transmitted from the first light receiving fiber 56, the second light receiving fiber 57, and the third light receiving fiber 58 can be detected with one piece of the imaging unit 23 alone. As a result, the optical measurement apparatus 500 can be downsized with the simple configuration.

### (Fourth Embodiment)

A fourth embodiment of the invention will now be described. An optical measurement apparatus according to the fourth embodiment differs in configuration of the imaging unit and the partition unit according to the second embodiment described above. Hence, a configuration of an imaging unit and a partition unit of the optical measurement apparatus will be described hereinafter. The same reference signs are used to designate the same elements as those in the embodiments described above.

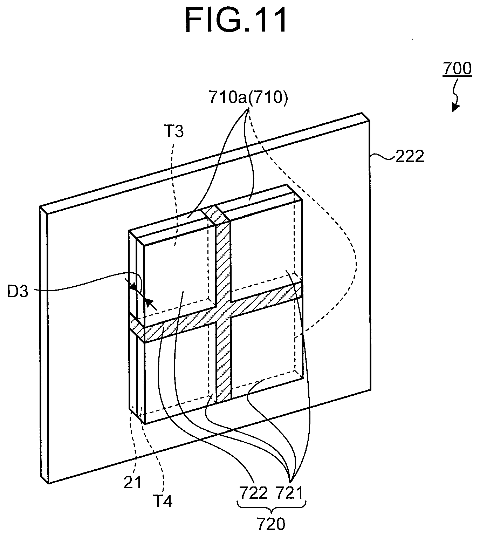

FIG. 11 is a schematic perspective view of a configuration of the imaging unit and the partition unit of the optical measurement apparatus according to the fourth embodiment of the invention. An optical measurement apparatus 700 illustrated in FIG. 11 includes an imaging unit 710 and a partition unit 720.

The imaging unit 710 includes a plurality of imaging elements 710a. The imaging elements 710a are each supported by a support member 222 with light reception surfaces T3 facing exit ends T1 of a measurement probe 400.

The partition unit 720 partitions the light reception surfaces T3, through which the imaging elements 710a each receive a ray of light, into a plurality, with the rays of light transmitted from the exit ends of a first light receiving fiber 56, a second light receiving fiber 57, and a third light receiving fiber 58 of the measurement probe 400 optically separated. Further, when the measurement probe 400 is inserted into an insertion portion 311a of the connector unit 310, the partition unit 720 sets a distance from the light reception surfaces T3 of the plurality of imaging elements 710a to the exit ends of the first light receiving fiber 56, the second light receiving fiber 57, and the third light receiving fiber 58 to a constant distance D3.

The partition unit 720 is realized by using transparent members and a light-blocking member, which have a plate shape and have the specified thickness D3. Specifically, the partition unit 720 is configured with a plurality of transparent members 721 such as glass and plastic, and a light-blocking member 722 for blocking light. The light-blocking member 722 is cross-shaped and provided between a transparent member 721 and a transparent member 721. The transparent members 721 and the light-blocking member 722 are integrated with an adhesive or the like. Alternatively, the partition unit 720 may be formed by integrating the transparent members 721 and the light-blocking member 722 through two-color molding.

In the fourth embodiment of the invention described above, the partition unit 720 partitions the light reception surfaces T3 of the plurality of imaging elements 710a into the plurality, with the rays of light transmitted from the exit ends T1 of the first light receiving fiber 56, the second light receiving fiber 57, and the third light receiving fiber 58 of the measurement probe 400 optically separated. The partition unit 720 also sets the distance from the exit ends T1 of the first light receiving fiber 56, the second light receiving fiber 57, and the third light receiving fiber 58 of the measurement probe 5 to the light reception surfaces T3 of the plurality of imaging elements 710a to the distance D3 when the measurement probe 400 is inserted into the insertion portion 311a of the connector unit 310. Thus, the optical measurement apparatus 500 can be downsized with the simple configuration.

### (First Modification of Fourth Embodiment)

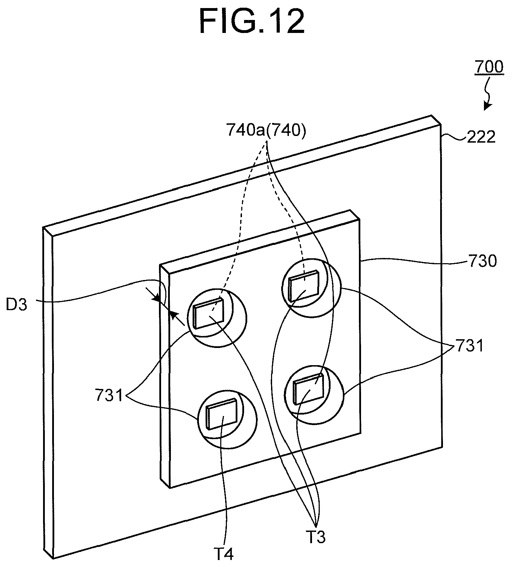

In the fourth embodiment of the invention, the shape of the imaging unit and the partition unit can be changed. FIG. 12 is a schematic perspective view of a configuration of an imaging unit and a partition unit according to a first modification of the fourth embodiment of the invention.

A partition unit 730 illustrated in FIG. 12 has holes 731 therethrough at positions corresponding to the exit ends T1 of the first light receiving fiber 56, the second light receiving fiber 57, and the third light receiving fiber 58 of the measurement probe 400 and the incoming end T2 of the illumination fiber 55. The partition unit 730 is realized by using a plate-shaped light-blocking member having a specified thickness D3.

An imaging unit 740 includes a plurality of imaging elements 740a. The imaging elements 740a are each supported by the support member 222 with light reception surfaces T3 facing the exit ends T1 of the measurement probe 400. The imaging elements 740a are each rectangular and accommodated in the holes 731 in the partition unit 730. Specifically, the imaging elements 740a are positioned in the paths for the rays of light transmitted from the exit ends T1 of the first light receiving fiber 56, the second light receiving fiber 57, and the third light receiving fiber 58 of the measurement probe 400.

The first modification of the fourth embodiment according to the invention as described above achieves a similar effect to the fourth embodiment described above and can downsize the optical measurement apparatus 700 with the simple configuration.

As described above, the invention can include various embodiments undescribed herein and allows various design modifications within the range of technical ideas defined by the scope of the claimed invention.

### Reference Signs List

- 1: Optical measurement system

- 2, 300, 500, 700: Optical measurement apparatus
- 3: Display unit
- 4: Input unit
- 5, 400: Measurement probe
- 20: Power source unit
- 21: Light source unit
- 22, 310, 600: Connector unit
- 23, 710, 740: Imaging unit
- 24, 320, 330, 340, 350, 720, 730: Partition unit
- 25: Recording unit
- 26: Control unit
- 51: Proximal end portion
- 52: Flexible portion
- 53: Distal end portion
- 54: Rod lens
- 55: Illumination fiber
- 56: First light receiving fiber
- 57: Second light receiving fiber
- 58: Third light receiving fiber
- 59, 401: Holding portion
- 60: Pressing member
- 221, 311: Connector frame
- 221a, 311a: Insertion portion
- 221b: First groove portion
- 221c: Second groove portion
- 221d: Abutment portion
- 222: Support member
- 233: Screw
- 241, 321: First partition unit
- 242: Second partition section
- 261: Calculation unit
- 331, 721: Transparent member
- 332, 722: Light-blocking member
- 341, 351, 731: Hole
- 601: Seat
- 602: Energizing member
- 710a, 740a: Imaging element
- T1: Exit end
- T2: Incoming end
- T3: Light reception surface
- T4: Light emission surface

## Claims

1. An optical measurement apparatus to which a measurement probe is configured to be detachably attached, the measurement probe having at an end surface thereof exit ends of a plurality of optical fibers, the optical measurement apparatus comprising:
an imaging unit configured to receive rays of light transmitted from the exit ends of the plurality of optical fibers and to perform photoelectric conversion in order to generate and output an electric signal; and
a partition unit configured to optically isolate paths for the rays of light transmitted from the exit ends of the plurality of optical fibers, from one another in a space from the end surface to a light reception surface of the imaging unit.

2. The optical measurement apparatus according to claim 1, wherein the partition unit comes into contact with the end surface at one end of the partition unit, and comes into contact with the light reception surface of the imaging unit at another end of the partition unit.

3. The optical measurement apparatus according to claim 1, further comprising a connector unit into which the measurement probe is configured to be inserted, wherein
the partition unit is provided between the connector unit and the imaging unit, and keeps a constant distance between the end surface and the light reception surface when the measurement probe is inserted into the connector unit.

4. The optical measurement apparatus according to claim 1, further comprising:
a support member configured to support the imaging unit; and
an energizing member configured to energize the support member toward the end surface.

5. The optical measurement apparatus according to claim 1, wherein
the imaging unit includes a plurality of imaging elements, and
the plurality of imaging elements is provided on the paths for the rays of light isolated by the partition unit.

6. The optical measurement apparatus according to claim 1, further comprising a connector unit into which the measurement probe is configured to be inserted, the connector unit keeping a constant distance between the end surface and the light reception surface when the measurement probe is inserted.
